Europäisches Patentamt

European Patent Office

Office européen des brevets

(11) Veröffentlichungsnummer: **0 041 611**
A2

# (12) EUROPÄISCHE PATENTANMELDUNG

(21) Anmeldenummer: 81103323.2

(22) Anmeldetag: 02.05.81

(51) Int. Cl.³: **A 61 K 6/04**

(30) Priorität: 14.05.80 DE 3018376

(43) Veröffentlichungstag der Anmeldung:
16.12.81 Patentblatt 81/50

(84) Benannte Vertragsstaaten:
BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Rehberg, Hans-Joachim, Prof. Dr.
Nobelstrasse 33
D-5090 Leverkusen 1(DE)

(54) Metallische Pulvermischungen zur Anfertigung zahnärztlicher Amalgame.

(57) Metallische Pulvermischung zur Anfertigung eines zahnärztlichen Amalgams, bestehend aus
a) einem Silber, Zinn und gegebenenfalls weitere Metalle
enthaltenden Legierungspulver und
b) einem feinen, lamellaren Pulver aus Kupfer oder bis zu 75
% andere Metalle enthaltenden kupferhaltigen Legierungen,
die als Füllungsmaterial für Zähne gebraucht werden.

EP 0 041 611 A2

BAYER AKTIENGESELLSCHAFT     5090 Leverkusen, Bayerwerk
Zentralbereich
Patente, Marken und Lizenzen     Si/ABc     12. Mai 1980

Metallische Pulvermischungen zur Anfertigung zahnärztlicher Amalgame

Die Erfindung betrifft metallische Pulvermischungen
zur Anfertigung zahnärztlicher Amalgame, die als
Füllungsmaterial für Zähne gebraucht werden.

Zahnärztliche Amalgame werden hergestellt durch Vermischen von feinpulverisierten Metall-Legierungen
mit Quecksilber. Als Metall-Legierungen werden üblicherweise hauptsächlich Silber und Zinn enthaltende
Legierungen benutzt, die noch Zusatz von Kupfer,
Zink und Quecksilber enthalten können. In den bestehenden gültigen Normen für "Legierungen zum Herstellen zahnärztlicher Amalgame" sind die erlaubten
Legierungsanteile wie folgt näher begrenzt:

Die Legierung muß mindestens 65 % Silber und höchstens
29 % Zinn, höchstens 6 % Kupfer und höchstens 2 % Zink
sowie höchstens 3 % Quecksilber enthalten.

Le A 20 373-Ausland

Derartige Legierungen werden heute als "konventionelle" Legierungen bezeichnet und sind seit vielen Jahrzehnten im Handel.

Diese konventionellen, in Pulverform - hergestellt durch Verspanen oder Verdüsen - vorliegenden Legierungen werden üblicherweise im Gewichtsverhältnis von etwa 1 : 1 mit Quecksilber angemischt. Die Mischung bildet dann eine plastische zusammenhängende Masse, die vom Zahnarzt in die vorbereitete Kavität im Zahn eingebracht wird. Unmittelbar dabei und danach wird die Masse durch instrumentelle Druckanwendung kondensiert und erhärtet dann innerhalb weniger Minuten zu einer festen Masse.

Die Erhärtung der plastischen Mischung erfolgt bekannterweise durch Bildung zweier neuer kristalliner Phasen, nämlich einer Silber-Quecksilber-Phase (Gamma-1) und einer Zinn-Quecksilber-Phase (Gamma-2). In diesen Phasen sind eine Vielzahl von den im Überschuß zugegebenen Pulverteilchen eingebettet, die nur oberflächlich mit Quecksilber reagiert haben.

Man weiß, daß die Gamma-2-Phase nicht nur die mechanisch weichste Phase, sondern auch die korrosionsanfälligste Phase im erhärteten Amalgam ist, wodurch dessen physikalische und Korrosions-Eigenschaften sofort und auf die Dauer nachteilig beeinflußt werden können.

Le A 20 373

In letzter Zeit wurden daher viele Versuche durchgeführt, die Gamma-2-Phase in ihrem mengenmäßigen Anteil innerhalb des Amalgams zu vermindern oder sogar zu eliminieren. Hierzu eignen sich bekannterweise entweder bestimmte Zusätze zum üblichen Pulver oder Änderungen in der Pulver-Legierungszusammensetzung.

Im US-Patent 3 305 356 wird z.B. der Zusatz des Silber-Kupfer-Eutektikums (Cu-Gehalt ca. 28 %) erwähnt; dieses Eutektikum wird aus der Schmelze versprüht und das erhaltene Kugelpulver in einem Prozentsatz von etwa 30 % einem normalen konventionellen Legierungspulver beigemischt.

Im US-Patent 3 871 876 wird dagegen eine einheitliche Legierung geschützt, die mehr Kupfer als früher erlaubt und üblich, nämlich ca. 13 % Kupfer, enthält und als Pulver Anwendung findet.

In beiden Fällen ergeben die Pulver nach dem Vermischen mit Quecksilber Amalgame, die nach einigen Tagen praktisch keine Gamma-2-Phase enthalten.

Wie aus Mikroanalysen hervorgeht, verbindet sich während solcher Amalgamierungen ein Teil des Legierungskupfers mit dem vom Quecksilber gelösten Zinn, so daß es nicht zur Bildung der Zinn-Quecksilber-Phase kommt, sondern eine Kupfer-Zinn ($Cu_6Sn_5$)-Phase gebildet wird.

Wie aus den Mikroanalysen weiterhin hervorgeht, reagiert aber z.B. bei der Pulvermischung nach US-Patent 3 305 356 nur eine äußerst dünne äußere Schale der kupferhaltigen Eutektikum-Kugeln, in der sich $Cu_6Sn_5$ bildet.

In den einheitlichen Legierungspulvern entsprechend dem US-Patent 3 871 876, liegt das Kupfer, wie Mikroanalysen zeigen, an bestimmten Stellen eines Pulverteilchens als $Cu_3Sn$ angehäuft. Daher reagieren bei diesem Pulver - nach dem Vermischen mit Quecksilbernur diejenigen kupferhaltigen äußeren Stellen eines Pulverteilchens, die vom Quecksilber erreicht werden, die dann mit Zinn wieder $Cu_6Sn_5$ bilden.

Es ist weiterhin bekannt, daß sich reines Kupfer nur äußerst schwierig mit Quecksilber benetzt und erst nach Wochen zur Bildung von Kupferamalgam ($Cu_7Hg_6$) führt. Wie z.B. aus der Dissertation von HÄSSELBARTH (Freie Universität Berlin 1958) hervorgeht, benötigt selbst die Benetzung eines Kupferpulvers mit Quecksilber beim Anreiben unter Druck mehrere Stunden. Eigene Versuche bestätigen dies.

Aus der DE-OS 26 15 346 ist ein Amalgam für zahnärztliche Zwecke bekannt, wobei das Quecksilber mit einer Silber/Zinn/Kupfer/Zink-Legierung vermischt wird, welche einen Ag-Gehalt von etwa 65 Gew.-%, einen Sn-Gehalt von etwa 29 Gew.-%, einen Cu-Gehalt von bis zu etwa 6 Gew.-% und einen Zn-Gehalt von bis zu etwa 2 Gew.-% aufweist und mit einem Zusatz versehen ist, welcher da-

Le A 20 373

durch gekennzeichnet ist, daß als Zusatz elementares Kupfer oder eine Kupferlegierung vorgesehen ist. Auf Seite 5 dieser Offenlegungsschrift ist angegeben, daß das zugesetzte elementare Kupfer entweder span- oder kugelförmige Gestalt aufweisen können. Elementares Kupfer reagiert jedoch in einer solchen Form praktisch nicht. Auch die Angaben der Tabelle II der Offenlegungsschrift erscheinen fragwürdig, denn weder im Text noch in der Tabelle wird die Methode der $\gamma_2$-Bestimmung genannt. Die einzige wissenschaftlich eindeutige Methode ist die Kristallphasen-Bestimmung mit einem Röntgen-Goniometer, das qualitative und quantitative Aussagen gestattet. Allerding liegt die Nachweisgrenze für derartige Phasen bei einem Gehalt von ca. 1 %.

Überraschenderweise wurde nun gefunden, daß sich ein feines, lamellares Pulver aus Kupfer (andere Elemente werden für die Bildung der $Cu_6Sn_5$-Phase nicht benötigt) mit Quecksilber leicht und schnell anmischen läßt, wenn es vorher mit einem konventionellen Legierungspulver gemischt wurde. Es resultiert ene plastische Mischung, die wie gewohnt nach wenigen Minuten zu einem harten Amalgam erhärtet, das wenig oder keine Gamma-2-Phase (Zinn-Quecksilber) aufweist.

Gegenstand der Erfindung sind daher metallische Pulvermischungen zur Anfertigung zahnärztlicher Amalgame, bestehend aus

a) einem hauptsächlich Silber, Zinn und gegebenenfalls weitere Metalle enthaltenden Legierungspulver (Späne oder Kugeln) und

Le A 20 373

b)  einem feinen, lamellaren Pulver aus Kupfer oder
kupferhaltigen Legierungen.

Ein Vorteil des erfindungsgemäßen Zusatzes an feinem
lamellaren Kupfer ist darin zu sehen, daß es
gegenüber den sonst üblichen Zusätzen von kugeligen,
hoch kupferhaltigen Legierungspulvern eine wesentlich
größere Oberfläche für die beabsichtigte Reaktion zu
$Cu_6Sn_5$ aufweist. Es reichen daher schon relativ geringe
Kupfermengen d. h. solche zwischen 1 und 10 % Kupferpulver in Abhängigkeit von der Zusammensetzung der
"konventionellen Legierung", um die Menge an Gamma-2-
Phase in erhärtetem Amalgam deutlich zu vermeiden bzw.
deren Entstehung zu verhindern.

Der pulverförmige Zusatz b) muß nicht aus reinem Kupfer
bestehen, sondern kann auch eine kupferhaltige Legierung,
mit Silber und/oder mit Zinn, sein. Auch andere kupferhaltige Legierungen mit Zink und anderen Metallen sind
anwendbar, wenn sie sich als feines, lamellares Pulver
herstellen lassen und wenn sie nach Zusatz zu einem
konventionellen Legierungspulver, die Bildung der Gamma-2-
Phase verhindern oder deutlich vermindern.

Diese Bedingungen sind dann erfüllt, wenn die Kupferlegierungen bis zu 75 % der genannten anderen Metalle
enthalten.

Das erfindungsgemäße feine, lamellare Pulver
aus Kupfer bzw. Kupferlegierungen besteht aus Blättchen
mit einer Dicke von 0,01 bis 2 /um, vorzugsweise von

0,1 bis 1 $\mu$m, die eine unregelmäßige begrenzte freie Oberfläche von ca. 10 bis 50 000 $\mu$m$^2$, vorzugsweise zwischen ca. 1000 und 30 000 $\mu$m$^2$, aufweisen. Ein solches Kupferpulver liefert die Carl Schlenk AG, Nürnberg unter der Bezeichnung "Schlenk Kupferpulver TT30 natur". Unter dem Begriff "freie Oberfläche" soll die Oberfläche auf beiden Seiten des Blättchens verstanden werden.

Nur bei der erfindungsgemäß verwendeten lamellaren Form der zugesetzten Kupfer- oder Legierungspartikeln in Mengen bis zu 10 % wird der gewünschte Effekt der deutlichen Verringerung der $\gamma_2$-Phase erreicht. Demgegenüber bewirkt gemäß DE-OS 26 15 346 der 7 %ige Zusatz einer Legierung aus 54 % Kupfer und 46 % Silber nur eine kleine Verringerung von 10 % $\gamma_2$-Phase auf 7,2 %. Eine ebenfalls nur minimale Verringerung der $\gamma_2$-Phase tritt auch dann ein, wenn anstelle des erfindungsgemäß verwendeten lamellaren Kupfers z.B. kugelförmige Kupferpulverteilchen verwendet werden.

Le A 20 373

Beispiel:

Unter ein feinzerspantes konventionelles Legierungspulver aus 67 % Silber, 27 % Zinn, 4 % Kupfer, 1,5 % Zink und 1 % Quecksilber werden unterschiedliche Mengen eines feinen, lamellaren Kupferpulvers gründlich untergemischt. Es werden hierdurch 3 verschiedene Pulvermischungen erhalten, die 3 %, 7 % und 10 % Kupferpulver enthalten.

Von dem konventionellen Legierungspulver und von jeder Pulvermischung werden sodann Mischungen mit Quecksilber im Anmischverhältnis 0,9:1 in einem handelsüblichen Amalgammischer (Duomat) hergestellt.

Es wird jedesmal eine plastische Masse erhalten, die entsprechend der Prüfkörper-Herstellungsvorschrift der ADA-Spezifikation Nr. 1 in eine zylinderförmige Hohlform gestopft und kondensiert wird. Nach dem Erhärten der Körper werden diese aus der Hohlform entnommen und zwei Tage bei 37°C gelagert. Diese Formkörper werden anschließend für eine metallographische Untersuchung eingebettet, geschliffen und poliert.

Mit Hilfe der Röntgenbeugung in einem Goniometer werden nun die Prüfkörper auf ihren Gehalt an Gamma-2-Phase vergleichend untersucht. Die Intensität der Röntgenstrahlbeugung am Kristallgitter der Gamma-2-Phase gibt dann Aufschluß über deren Anteil im Gefüge des Prüfkörpers.

Le A 20 373

- 9 -

Wie die nachstehende Tabelle, in der a) den Anteil an
konventioneller Legierung und b) den Anteil an feinem,
lamellaren Zusatz von Kupfer oder kupferhaltiger
Legierung bedeutet, zeigt, nimmt die Intensität für
die Gamma-2-Phase mit steigenden Anteilen von Kupfer
in der Pulvermischung ab. Der Gehalt an Gamma-2-Phase,
entsprechend der Beugungsintensität, bei der Probe
mit 0 % - Kupferzusatz wurde für diesen Vergleich gleich
100 % gesetzt. Wie ersichtlich, liegt bereits ab einem
Zusatz von 7 % Kupferpulver der Anteil an Gamma-2-Phase
unter der Nachweisgrenze. Gleichzeitig steigt der Anteil an der neu gebildeten $Cu_6Sn_5$-Phase.

Tabelle

| a) | b) | rel.Gehalt an Gamma-2* | rel.Intensität $Cu_6Sn_5$ |
|---|---|---|---|
| 100 % | 0 % | 100 % | 35 |
| 97 % | 3 % | 30 % | 95 |
| 93 % | 7 % | <10 %** | 210 |
| 90 % | 10 % | <10 %** | 230 |

\* nicht tatsächlicher Gehalt, sondern vergleichende
  Angaben

\*\* Nachweisgrenze wegen zu geringer relativer Intensität
  erreicht; tatsächlicher Gamma-2-Gehalt unter 1 %.

Le A 20 373

Damit ist erwiesen, daß das Kupfer während der
Amalgamierung in die Reaktion mit einbezogen wurde,
denn es hat sich anstelle der Zinn-Quecksilber-Verbindung die Kupfer-Zinn-Verbindung in steigendem Anteil
neu gebildet. Dies wäre nicht möglich gewesen, wenn das
Quecksilber die Oberfläche der Kupfer-Pulverteilchen
nicht benetzt hätte.

Le A 20 373

Patentansprüche:

1. Metallische Pulvermischung zur Anfertigung eines
   zahnärztlichen Amalgams, bestehend aus
   a) einem Silber, Zinn und gegebenenfalls weitere
      Metalle enthaltenden Legierungspulver und
   b) einem feinen, lamellaren Pulver aus Kupfer
      oder bis zu 75 % anderen Metalle enthaltenden
      kupferhaltigen Legierungen.

2. Metallische Pulvermischung gemäß Anspruch 1, dadurch
   gekennzeichnet, daß das Legierungspulver gemäß a) in
   Spanform vorliegt.

3. Metallische Pulvermischung gemäß Anspruch 1, dadurch gekennzeichnet, daß das Legierungspulver gemäß a) in Kugelform vorliegt.

4. Metallische Pulvermischung gemäß Anspruch 1, dadurch gekennzeichnet, daß sie 1 bis 15 % des feinen lamellaren
   Pulvers aus Kupfer oder kupferhaltigen Legierungen enthält.

5. Metallische Pulvermischung gemäß Anspruch 1, dadurch
   gekennzeichnet, daß das feine, lamellare Pulver aus
   Kupfer besteht.

6. Metallische Pulvermischung gemäß Anspruch 1, dadurch gekennzeichnet, daß das feine, lamellare Pulver aus
   einer Legierung aus Kupfer und bis zu 75 % Silber besteht.

7. Metallische Pulvermischung gemäß Anspruch 1, dadurch gekennzeichnet, daß das feine, lamellare Pulver aus einer
   Legierung aus Kupfer und bis zu 30 % Zinn besteht.

Le A 20 373

0041611

8. Metallische Pulvermischung gemäß Anspruch 1, dadurch gekennzeichnet, daß das feine, lamellare Pulver aus einer Legierung aus Kupfer und bis zu insgesamt 75 % Silber und Zinn besteht.

9. Metallische Pulvermischung gemäß Anspruch 1, dadurch gekennzeichnet, daß das lamellare Kupfer- bzw. Kupferlegierungspulver, in einer Dicke je Einzelblättchen zwischen 0,01 $\mu$m und 2 $\mu$m, vorzugsweise zwischen 0,1 und 1 $\mu$m, vorliegt.

10. Metallische Pulvermischung gemäß Anspruch 1, dadurch gekennzeichnet, daß das lamellare Kupfer- bzw. Kupferlegierungspulver mit einer freien Oberfläche von ca. 10 bis 50 000 $\mu$m$^2$, vorzugsweise zwischen 1000 bis 30 000 $\mu$m$^2$, vorliegt.

Le A 20 373